**Europäisches Patentamt**

**European Patent Office**

**Office européen des brevets**

(19)

(11) Veröffentlichungsnummer: **0 033 724**

**A2**

(12) **EUROPÄISCHE PATENTANMELDUNG**

(21) Anmeldenummer: **81890017.7**

(22) Anmeldetag: **28.01.81**

(51) Int. Cl.³: **C 12 M 1/00**
**C 12 P 5/02**

(30) Priorität: **30.01.80 AT 476/80**

(43) Veröffentlichungstag der Anmeldung:
**12.08.81 Patentblatt 81/32**

(84) Benannte Vertragsstaaten:
**CH DE FR GB IT LI**

(71) Anmelder: **Ing. Leibetseder Gesellschaft m.b.H. & Co. KG.**
**Pestalozzistrasse 7**
**A-4300 St. Valentin(AT)**

(72) Erfinder: **Leibetseder, Ferdinand**
**Pestalozzistrasse 7**
**A-4300 St. Valentin(AT)**

(74) Vertreter: **Hübscher, Gerhard, Dipl.-Ing. et al,**
**Patentanwälte Dipl.-Ing. Gerhard Hübscher Dipl.-Ing.**
**Helmut Hübscher Dipl.-Ing. Heiner Hübscher**
**Spittelwiese 7**
**A-4020 Linz(AT)**

(54) **Gärbehälter für die anaerobe Gärung faulfähiger Substanzen.**

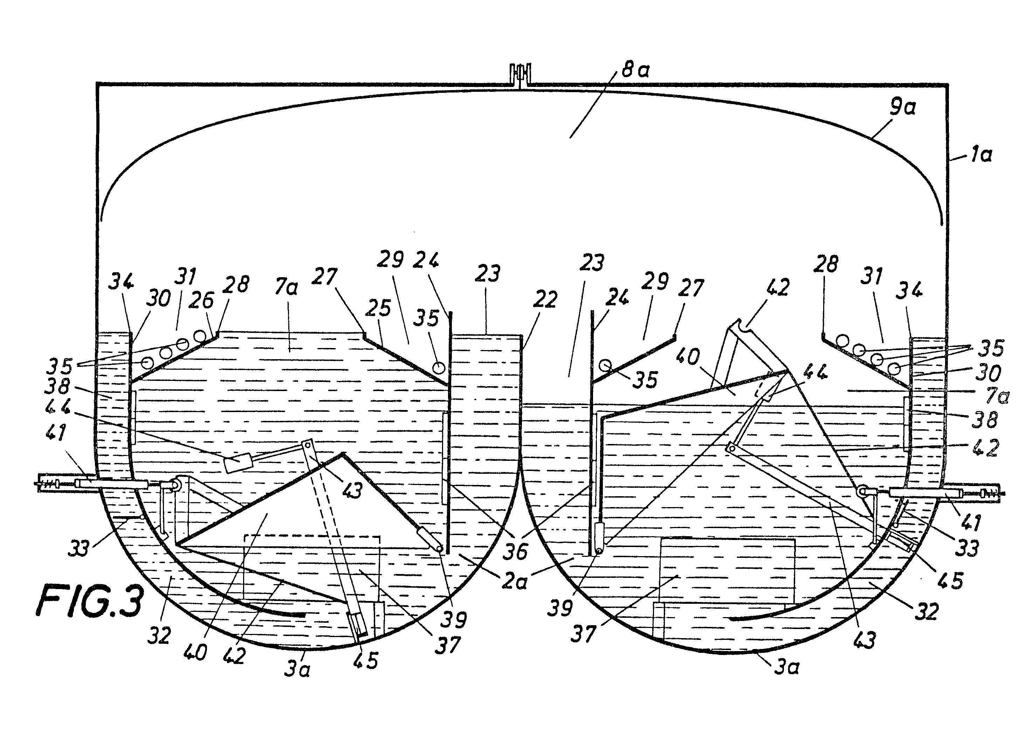

(57) Ein Gärbehälter für die anaerobe Gärung besitzt eine Faulkammer (7, 7a) mit Einlässen und Auslässen für eine faulfähige Substanz und eine Gaskammer (8, 8a) für das erzeugte Gas. Zur Erzielung einer weitgehenden Wartungsfreiheit werden das entstehende Gas selbst oder von ihm erzeugte Kräfte zur Zerstörung einer Schwimmschicht und zur Entfernung von Rückständen aus der Faulkammer ausgenützt. Dafür ist ein mit der Faulkammer (7, 7a) kommunizierender Sammelraum (6, 40) vorgesehen, in den zunächst faulfähige Substanz eindringen kann, der während der Gärung einen Teil des entstehenden Gases aufnimmt und dessen Gasraum periodisch in die Gaskammer (8, 8a) entleerbar ist, wobei die faulfähige Substanz in Bewegung versetzt wird. Dafür ist entweder ein Absperrorgan (17) an einem Auslaß des Sammelraumes (6) vorgesehen oder der Sammelraum bildet in der Faulkammer (7a) eine aus einer Sammelstellung für das Gas in eine Entleerungsstellung und zurück verstellbare Taucherglocke (40). (Fig. 3)

FIG.3

EP 0 033 724 A2

Gärbehälter für die anaerobe Gärung faulfähiger
Substanzen

Die Erfindung betrifft einen Gärbehälter für die anaerobe Gärung faulfähiger Substanzen, insbesondere zur Erzeugung von Biomethangas, mit einer Ein- und Auslässe für die faulfähige Substanz aufweisenden Faulkammer und einer Gaskammer für das entstehende Gas.

Bekanntlich setzt die anaerobe Gärung faulfähiger Substanzen, wie von Tiergülle und Pflanzenresten, Biomethangas frei, wobei der nutzbare Anteil dieses Gases, das Methan, wie Erdgas verwendet verwendet werden kann. Eine anaerobe Gärung kann aber auch vorwiegend dazu verwendet werden, um Gülle und Pflanzenreste rasch zu einem brauchbaren Dünger aufzubereiten.

In der Praxis ergibt sich bei bekannten Gärbehältern das Problem, daß sich in der Faulkammer auf der meist in Form einer Aufschwemmung vorliegenden faulfähigen Substanz eine Schwimmdecke bildet, die die Gärung und damit die Gasentwicklung hemmt. Es ist zwar möglich, zur Beseitigung dieser Schwimmdecke Rührwerke einzusetzen, doch hat dies nicht immer den gewünschten Erfolg, wobei noch zu bedenken ist, daß für den Antrieb der Rührwerke Energie eingesetzt werden muß, so daß sich die Gesamtenergiebilanz bei der Biomethangaserzeugung verschlechtert. Sonst muß der Gärbehälter von Zeit zu Zeit geöffnet und die Schwimmdecke entfernt werden, was eine unangenehme Arbeit darstellt und vor allem praktisch nur unter Luftzutritt zur Faulkammer durchgeführt werden kann, so daß die Gärung unterbrochen wird bzw. die Gefahr des Entweichens wenigstens eines Teiles des erzeugten Biomethangases besteht. Bei vielen faulfähigen Substanzen kommt es ferner zu einem Absetzen schwererer Stoffe am Boden des Gärbehälters, so daß im Laufe der Zeit Verkrustungen entstehen, die nach vorherigem Entleeren des Behälters durch Abkratzen entfernt werden müssen. Auch hier kommt es zu einer Unterbrechung des Betriebes. Der Gärbetrieb selbst hängt in seinem Verlauf von den jeweils zur Verfügung stehenden Substanzen ab, deren Zusammensetzung bzw. Gärfähigkeit sich sogar bei der Verwendung von Gülle u.a. in Abhängigkeit vom jeweiligen Tierbestand und der Art der Fütterung ändern kann. Mit bekannten Gärbehältern kann der Gärprozeß nur wenig beeinflußt werden bzw. ist es nicht möglich, eine ausreichende Anpassung an verschiedene zu vergärende Substanzen zu erzielen, so daß häufig diese Substanzen vor einer ausreichenden Vergärung aus dem Behälter entnommen werden, was nicht nur den Wirkungsgrad verschlechtert, sondern auch wegen der unvollständigen Gärung zu einer starken Geruchsbelästigung führt. Alle diese Gründe ergeben, daß bisher die Biomethangaserzeugung auf Versuchsanlagen und Großanlagen beschränkt ist.

Aufgabe der Erfindung ist die Schaffung eines Gärbehälters, der bei einfachem Aufbau weitgehend wartungsfrei betrieben werden kann, bei dem eine Anpassung an den jeweiligen Verlauf der Gärung möglich ist und bei dem eine Aufrechterhaltung des Gärbetriebes über lange Zeiträume gewährleistet werden kann.

Die gestellte Aufgabe wird prinzipiell dadurch gelöst, daß die Faulkammer einen mit einem Auslaß verbindbaren Überlauf aufweist und mit ihr ein unter dem vom Überlauf bestimmten Niveau angeordneter, nach unten offener Sammelraum kommuniziert, der bei Chargenbeginn zumindest zum Großteil mit der faulfähigen Substanz gefüllt ist, wenigstens einen Teil des in seinem Bereich entstehenden Gases aufnimmt und dessen über der mit zunehmender Gasfüllung verdrängten faulfähigen Substanz entstehender Gasraum periodisch in die Gaskammer entleerbar ist, so daß die faulfähige Substanz durchmischt und eine Schwimmschicht zerstört bzw. durch die entstehende Strömung und das Nachströmen faulfähiger Substanz von einem Vorrat her am Überlauf abgeschieden wird.

Der Grundgedanke der Erfindung besteht darin, die Auftriebskraft oder das Volumen des entstehenden Biomethangases weitgehend unmittelbar für eine Durchmischung bzw. Weiterförderung der faulfähigen Substanz im Gärbehälter auszunützen und eine allenfalls gebildete Schwimmdecke zu zerstören bzw. zum Überschwappen zu bringen, so daß die bisher notwendigen Rührwerke wegfallen können und überdies der Gärbetrieb durch keine zu starke Schwimmschicht beeinträchtigt wird. Man muß lediglich dafür Sorge tragen, daß der Fülldruck am Einlauf des Gärbehälters ausreicht, um ein ausreichendes Nachströmen der Gärsubstanz bis zum jeweiligen Füllniveau zu gewährleisten. Ob man den Fülldruck durch ein entsprechendes Gefälle in einem Zubringerkanal oder -schacht oder durch Pumpen erzeugt, ist gleichgültig.

Im Rahmen der Erfindung sind verschiedene Varianten der Grundkonstruktion möglich. Nach einer dieser Varianten ist der Sammelraum ortsfest angeordnet und kommuniziert über eine unter dem Füllniveau liegende Verbindung mit der Faulkammer, wobei der Sammelraum selbst einen mit dem Auslaß verbundenen Überlauf bestitzt, der zugleich den zur Gaskammer führenden Gasauslaß bildet, über ein Absperrorgan verschließbar und auf einem niedrigeren Niveau als der Überlauf der Faulkammer angeordnet ist, so daß der bei geschlossenem Absperrorgan im Sammelraum entstehende Gasdruck die faulfähige Substanz in die Faulkammer und schließlich zu deren Überlauf drängt, wobei eine Schwimmschicht abgeschieden wird, wogegen beim Öffnen des Absperroganes nach dem Entweichen des gebildeten Gases in die Gaskammer bis zum Schließen des Absperrorganes ein Überlauf stattfinden kann, so daß auch aus dem Sammelraum eine allfällige Schwimmschicht entfernt wird.

Das Abscheiden der Schwimmschichten des Sammelraumes und der Faulkammer erfolgt zu verschiedenen Zeiträumen und es befindet sich daher wechselweise der Sammelraum und die Faulkammer im günstigsten Gärbetrieb.

Konstruktiv ergibt sich eine besonders einfache Ausführung dadurch, daß die Überläufe als auf verschiedenen Höhen an einem gemeinsamen Fallschacht zur Ableitung des Überlaufgutes liegende Überlaufkanten des Sammelraumes und der Faulkammer und das Absperrorgan als Klappe ausgebildet sind.

Bei der beschriebenen Konstruktionsvariante wird der Gasdruck im Sammelraum für die Beseitigung der Schwimmschichten ausgenützt, so daß kein zusätzlicher Energieaufwand erforderlich ist. Lediglich zur Betätigung des Absperrorganes könnte eine äußere Energiequelle vorgesehen werden, doch ist hier der Energieaufwand sehr gering.

Zur Steuerung des Absperrorganes können Zeitschaltwerke und bzw. oder Fühler vorgesehen sein, die auf bestimmte Betriebszustände (Niveau, Druck, Temperatur) im Sammelraum bzw. der Faul- oder Gaskammer ansprechen.

- 5 -

Durch Wahl des Volumsverhältnisses von Sammelraum und Faulkammer und durch Festlegung des Füllungszustandes des Sammelraumes, bei dem das Gärgas aus ihm abgelassen wird, kann man den durchschnittlichen Gärungsverlauf bestimmen bzw. an die jeweilige faulfähige Substanz anpassen. Die Füllhöhen im Sammelraum und in der Faulkammer können durch Fühler, z.B. Schwimmer, überwacht werden. Wenn beim Öffnen des Absperrorganes bei der zuletzt beschriebenen Konstruktion kein Rückströmen eines Teiles des Faulgutes aus der Faulkammer in den Sammelraum erwünscht ist, kann man in der Verbindung Rückschlagklappen vorsehen. Der Anschluß an den Faulgutzulauf erfolgt meist über einen Siphon. Auch hier könnte eine Rücklschlagklappe angebracht werden.

Damit die Schwimmschicht sicher abgeschieden wird, können die Faulkammer bzw. der Sammelraum schräg zum Überlauf ansteigende Leitflächen aufweisen.

Bei einer anderen konstruktiven Ausführung des erfindungsgemäßen Gärbehälters bildet der Sammelraum eine Taucherglocke in der Faulkammer und ist innerhalb der Faulkammer aus einer Sammelstellung durch die vom gesammelten Gas erzeugte Auftriebskraft nach oben in eine Entleerungslage verstellbar gelagert, so daß die Taucherglocke nach Abgabe des Gases in die Sammelstellung zurückkehrt. Meist wird die Taucherglocke aus der Sammelstellung in die Entleerungslage und zurück verschwenkbar gelagert. Durch die Bewegung der Taucherglocke innerhalb des Faulgutes und durch das austretende Gas wird eine kräftige Durchmischung des Faulgutes hervorgerufen und es wird eine Schwimmschicht zum Überschwappen gebracht. Die Taucherglocke kann sozusagen selbst den Fühler bilden, der angibt, bei welchem Füllungsgrad die Entleerung ihres Gasinhaltes erfolgen soll. Es ist dazu nur notwendig, das Gewicht z.B. durch Ballastgewichte abzustimmen oder eine Konstruktion vorzusehen, bei der die Taucherglocke in der Sammelstellung über eine hinsichtlich ihrer Rückhaltekraft einstellbare Schnappverriegelung gehalten ist.

Nach einer Weiterbildung ist die Taucherglocke mit einem zur Loslösung von Ablagerungen über den Boden der Faulkammer verstellbaren Kratzer antriebsverbunden. Man kann diesen Kratzer einfach hin- und hergehend mit der Taucherglocke antreiben, um den Behälterboden frei von gefährlichen Ablagerungen zu halten. Zusätzlich kann der Kratzer aber auch einen Förderer bilden, der die erwähnten Ablagerungen in einen in seinem Verstellweg angeordneten Auslaßschacht fördert. Dieser Auslaßschacht kann zu dem auch vom Überlauf beschickbaren Auslaß führen und auch in diesem Auslaßschacht kann eine Rückschlagklappe vorgesehen sein. Die Beschickung des gemeinsamen Auslasses vom Überlauf und vom Auslaßschacht aus ergibt eine konstruktiv einfache Lösung, bei der nach einer Weiterbildung der Auslaß eine neben dem Überlauf angeordnete Sammelrinne aufweist, die über verschließbare Ablässe, wie Rohre oder Öffnungen entleerbar ist. In dieser Rinne können sich Teile der abgeschiedenen Schwimmschicht mit den über den Auslaßschacht zugebrachten Ablagerungen mischen und nachfaulen. Schließt man die Auslässe ab, kommt es zu einer Füllung der Rinne und gegebenenfalls zu einem Rückströmen von Faulgut aus der Rinne in die Faulkammer. Auch durch mehr oder weniger starkes Verschließen des Auslasses kann somit der Gärungsverlauf beeinflußt und die durchschnittliche Verweilzeit des Faulgutes im Gärbehälter eingestellt werden.

Nach einer Weiterbildung sind mehrere Gärbehälter nach Art einer Batterie an einem gemeinsamen von einem Vorrat her beschickbaren Zulauf und gemeinsame Ablässe angeschlossen, wobei man danach trachten wird, die ver-

- 7 -

schiedenen Gärbehälter jeweils in verschiedenen Gärphasen zu betreiben, um eine im Durchschnitt im wesentlichen gleichbleibende Gaserzeugung zu erzielen. Man kann durch Aneinanderreihung mehrerer Gärbehälter auch die Kapazität einer bestehenden Anlage vergrößern.

Weitere Einzelheiten und Vorteile des Erfindungsgegenstandes gehen aus der nachfolgenden Zeichnungsbeschreibung hervor.

In der Zeichnung ist der Erfindungsgegenstand beispielsweise veranschaulicht. Es zeigen

Fig. 1 schematisiert im Schnitt einen erfindungsgemäßen Gärbehälter in einem typischen Betriebszustand,

Fig. 2 den Gärbehälter nach Fig. 1 in einem anderen typischen Betriebszustand und

Fig. 3 zwei Gärbehälter einer Gärbehälterbatterie in Betriebszuständen, die etwa analog zu jenen nach den Fig. 1 bzw. 2 sind.

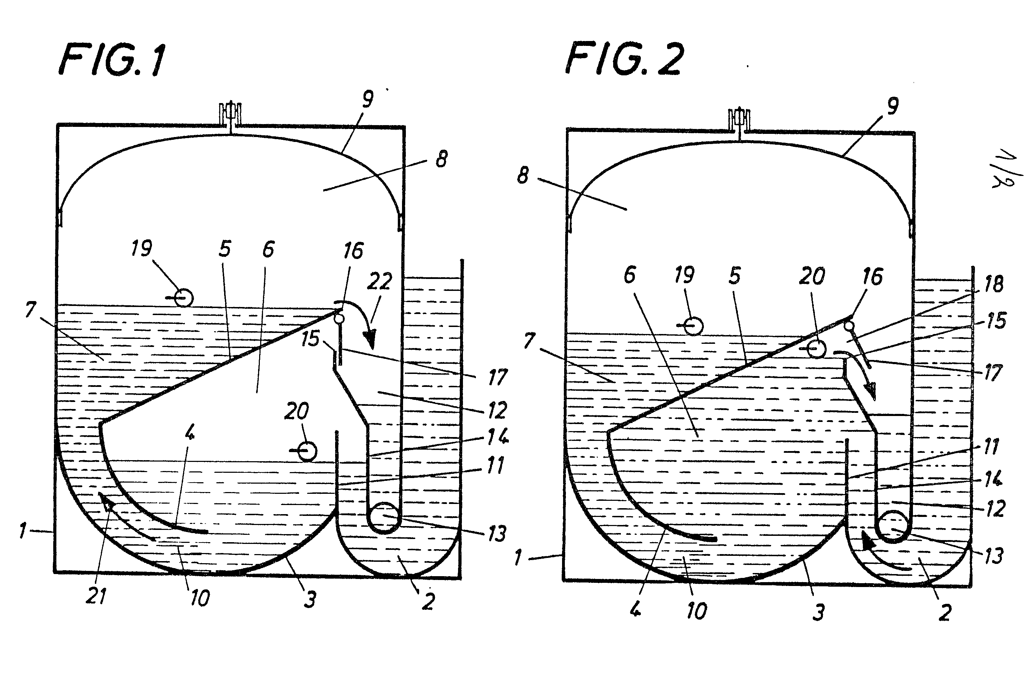

Der Gärbehälter nach den Fig. 1 und 2 besitzt ein Außengehäuse 1, das über einen einen Siphon 2 bildenden Einlaßkanal mit einer faulfähigen Substanz beschickbar ist. Durch eine gewölbte Bodenplatte 3, eine parallel dazu verlaufende Trennplatte 4 und eine schräg verlaufende Platte 5 wird der größere Teil des Innenraumes des Behälters in einen Sammelraum 6 und eine Faulkammer 7 unterteilt, wobei die Faulkammer 7 nach oben in eine Gaskammer 8 übergeht, für die beispielsweise eine membranartige, sich abhängig vom Gasdruck hebende und senkende bzw. sich aufblasende Decke 9 vorgesehen ist. Zwischen den Platten 3 und 4 wird ein den Sammelraum 6 mit der Faulkammer 7 verbindender Durchlaß 10 gebildet. Am Siphon 2 ist noch eine Trennwand 11 nach oben geführt. Innerhalb des vom Siphon 2 gebildeten Knies befindet sich ein Sammelschacht 12 mit Auslaß 13 für vergorenes Gut bzw. Material einer Schwimmdecke. Die eine Seitenwand 14 des Schachtes 12 bildet mit ihrer Oberkante 15 einen Überlaufrand für den Sammelraum 6 und die Oberkante 16 der Wand 5 bildet am Schacht 12 eine Überlaufkante der Faulkammer 7. Eine Klappe 17 verschließt eine obere Auslaßöffnung 18 des Sammelraumes 6. Schwimmer 19, 20 bewegen sich in Abhängigkeit vom jeweiligen Füllniveau des Sammelraumes 6 und der Faulkammer 7.

In Fig. 1 ist ein Betriebszustand dargestellt, der einige Zeit nach dem Schließen der Klappe 17 auftritt. Der Druck des im oberen Teil des Sammelraumes gesammelten Biomethangases drückt die faulfähige Substanz in Richtung des Pfeiles 21 aus dem Sammelraum 6 in die Faulkammer 7, so daß die Substanz schließlich über die Überlaufkante 16 ansteigt und eine Schwimmschicht bzw. die obere Schicht dieser faulfähigen Substanz aus der Faulkammer 7 entsprechend dem Pfeil 22 in den Schacht 12 gelangt.

Wird die Klappe 17 gemäß Fig. 2 geöffnet, dann kann das Gas aus dem Sammelraum 6 in den oberen Bereich des Schachtes 12 und von da in die Gaskammer 8 entweichen. Ein Teil der Substanz aus der Faulkammer 7 fließt über die Verbindung 10 in den Sammelraum 6 zurück. Über den Siphon 2 strömt frisches, faulfähiges Material nach, es kommt zu einer Durchmischung mit dem schon im Sammelraum 6 befindlichen bzw. in diesen zurückströmenden Material und der Flüssigkeitsspiegel im Sammelraum 6 steigt über das Niveau der Überlaufkante 15 an, so daß eine Schwimmschicht, die beim Ansteigen des Niveaus durch die Schrägwand 5 schon in Richtung auf den Überlauf abgelenkt wurde, schließlich über die Überlaufkante 15 hinweg in den Schacht 12 abgeschieden wird. Nach Schließen der Klappe 17 beginnt wieder der in Fig. 1 in einer Endphase dargestellte Zyklus.

Bei der Gärbehälterbatterie nach Fig. 3 wurden
der Konstruktion nach den Fig. 1 und 2 entsprechende
Teile mit den gleichen Bezugszeichen wie dort, die
aber den Zusatz a aufweisen, bezeichnet. Die beiden
Kammern sind gegengleich angeordnet und besitzen
beidseits einer Trennwand 22 verlaufende Einlaßkanäle
23, die an der anderen Seite von unter Bildung des
Siphons 2a vor der Bodenplatte 3a endenden Wänden 24
begrenzt sind. Schrägwände 25, 26 verkleinern die
freie Öffnung der jeweiligen Faulkammer und bilden
mit ihren oberen Enden Überlaufkanten 27, 28. Zwischen
der Wand 24 und der Schrägwand 25 wird eine Rinne 29
gebildet. Eine weitere Rinne wird von der Wand 26
und einer Zwischenwand 30 eingeschlossen, die ihrerseits einen Schacht 32 bestimmt, in dem eine Rückschlagklappe 33 angeordnet ist. Eine Überlaufkante 34 der
Wand 30 führt zur Rinne 31. Die Rinnen 29 und 31 bilden
Sammelrinnen und sind über für sich verschließbare
Auslaßöffnungen, Rohre od.dgl. 35 entleerbar. An den
Vorder- und Rückwänden und an den Wänden 24, 30 können
Heizkörper 36, 37, 38 zur Beschleunigung des Gärungsverlaufes angebracht sein.

In dem oberhalb des Siphons 2a liegenden Bereich
jeder Faulkammer 7a ist um eine Achse 39 eine einen
Sammelraum bildende Taucherglocke 40 schwenkbar gelagert,
die beim Ausführungsbeispiel die Grundform eines an der
längeren Wand offenen dreiseitigen Prismas besitzt. Diese
Taucherglocke 40 wird durch eine einstellbare Schnappverriegelung 41 in der in Fig. 3 links dargestellten
Lage gehalten. Sobald die Auftriebskraft in der Taucherglocke 40 durch die sich in ihr sammelnden Gase größer
wird als die Rückhaltekraft der Schnappverriegelung 41,
schwenkt die Glocke 40 nach Lösung der Verriegelung in
die in der rechten Kammer 3 a dargestellte Lage, so daß das Gärgas in den Sammelraum 8a entweichen kann, wonach die Taucherglocke 40 wieder in die Stellung nach Fig. 3 links zurückkehrt. Über einen Seilzug 42 ist mit der Taucherglocke 40 ein Winkelhebel 43 antriebsverbunden, der an dem einen Arm ein Gegengewicht 44 und am Ende des anderen Armes einen Kratzer 45 trägt, der über den Behälterboden 3a kratzt und Rückstände, die sich dort angesammelt haben, in den Schacht 32 fördert, so daß sie am Überlauf 34 in die Rinne 31 gelangen können. Die Rückschlagklappe 33 verhindert jeweils das Zurückströmen dieser Rückstände, wenn sich die Taucherglocke 40 und der Kratzer in die Ausgangslage nach Fig. 3 links zurückverstellen. Auch bei der Ausführung nach Fig. 3 können zur Niveauregelung und zur Erzeugung von Steuersignalen Schwimmer innerhalb der Taucherglocke und oben auf der Schwimmschicht vorgesehen sein.

Patentansprüche :

1. Gärbehälter für die anaerobe Gärung faulfähiger Substanzen, insbesondere zur Erzeugung von Biomethangas, mit einer Ein- und Auslässe für die faulfähige Substanz aufweisenden Faulkammer und einer Gaskammer für das entstehende Gas, dadurch gekennzeichnet, daß die Faulkammer (7, 7a) einen mit dem Auslaß (12, 29, 31) verbindbaren Überlauf (16, 27, 28) aufweist und mit ihr ein unter dem vom Überlauf bestimmten Niveau angeordneter, nach uten offener Sammelraum (6, 40) kommuniziert, der, bei Chargenbeginn zumindest zum Großteil mit der faulfähigen Substanz gefüllt ist, wenigstens einen Teil des in seinem Bereich entstehenden Gases aufnimmt und dessen über der mit zunehmender Gasfüllung verdrängten, faulfähigen Substanz entstehender Gasraum periodisch in die Gaskammer (8, 8a) entleerbar ist, so daß die faulfähige Substanz durchmischt und eine Schwimmschicht zerstört bzw. durch die entstehende Strömung und das Nachströmen faulfähiger Substanz von einem Vorrat her am Überlauf abgeschieden wird.

2. Gärbehälter nach Anspruch 1, dadurch gekennzeichnet, daß der Sammelraum (6) über eine unter dem Füllniveau liegende Verbindung (10) mit der Faulkammer (7) kommuniziert und selbst einen mit dem Auslaß (12) verbundenen Überlauf (15) besitzt, der zugleich den zur Gaskammer (8) führenden Gasauslaß bildet, über ein Absperrorgan (17) verschließbar und auf einem niedrigeren Niveau als der Überlauf (16) der Faulkammer (7) angeordnet ist, so daß der bei geschlossenem Absperrorgan (17) im Sammelraum entstehende Gasdruck die faulfähige Substanz in die Faulkammer (7) und schließlich zu deren Überlauf (17) drängt, wobei eine Schwimmschicht abgeschieden wird, wogegen beim Öffnen des Absperrorganes (17) nach dem Entweichen des gebildeten Gases in die Gaskammer (8) bis zum Schließen des Absperrorganes an dem ihm zugeordneten Überlauf ein Austritt einer allfälligen Schwimmschicht aus dem Sammelraum stattfindet.

3. Gärbehälter nach Anspruch 2, dadurch gekennzeichnet, daß die Überläufe als auf verschiedenen Höhen an einem gemeinsamen Fallschacht (12) zur Ableitung des Überlaufgutes liegende Überlaufkanten (15, 16) des Sammelraumes (6) und der Faulkammer (7) und das Absperrorgan als Klappe (17) ausgebildet sind.

4. Gärbehälter nach einem der Ansprüche 1 bis 3, dadurch gekennzeichnet, daß zur Steuerung des Absperrorganes (17) Zeitschaltwerke vorgesehen sind.

5. Gärbehälter nach einem der Ansprüche 1 bis 4, dadurch gekennzeichnet, daß zur Steuerung des Absperrorganes Fühler (19, 20) vorgesehen sind, die auf bestimmte Betriebszustände (Niveau, Druck, Temperatur) im Sammelraum bzw. der Faul- oder Gaskammer ansprechen.

6. Gärbehälter nach einem der Ansprüche 1 bis 5, dadurch gekennzeichnet, daß die Faulkammer (7a)bzw. der Sammelraum (6) schräg zum Überlauf (15, 27, 28) ansteigende Leitflächen (5, 25, 26) aufweisen.

7. Gärbehälter nach einem der Ansprüche 1 und 4 bis 6, dadurch gekennzeichnet, daß der Sammelraum eine Taucherglocke (40) in der Faulkammer (7a) bildet und innerhalb der Faulkammer aus einer Sammelstellung durch die vom gesammelten Gas erzeugte Auftriebskraft nach oben in eine Entleerungslage verstellbar gelagert ist, so daß die Taucherglocke nach Abgabe des Gases in die Sammelstellung zurückkehrt.

8. Gärbehälter nach Anspruch 7, dadurch gekennzeichnet, daß die Taucherglocke (40) aus der Sammelstellung in die Entleerungslage und zurück verschwenkbar gelagert ist.

9. Gärbehälter nach den Ansprüchen 7 und 8, dadurch gekennzeichnet, daß die Taucherglocke (40) in der Sammelstellung über eine hinsichtlich ihrer Rückhaltekraft einstellbare Schnappverriegelung (41) gehalten ist.

10. Gärbehälter nach Anspruch 7, dadurch gekennzeichnet, daß die Taucherglocke (40) mit einem zur Loslösung von Ablagerungen über dem Boden (3a) der Faulkammer verstellbaren Kratzer (45) antriebsverbunden ist.

11. Gärbehälter nach Anspruch 10, dadurch gekennzeichnet, daß der Kratzer (45) in einen in seinem Verstellweg angeordneten Auslaßschacht (32) fördert.

12. Gärbehälter nach Anspruch 11, dadurch gekennzeichnet, daß der Auslaßschacht (32) zu dem auch vom Überlauf (28) beschickten Auslaß (31) führt und im Auslaßschacht eine Rückschlagklappe (33) vorgesehen ist.

13. Gärbehälter nach einem der Ansprüche 1 bis 12, dadurch gekennzeichnet, daß der Auslaß eine neben dem Überlauf (27, 28, 34) angeordnete Sammelrinne (29, 31) aufweist, die über verschließbare Ablässe (35) entleerbar ist.

14. Gärbehälter nach einem der Ansprüche 1 bis 3, dadurch gekennzeichnet, daß mehrere Gärbehälter (7a) nach Art einer Batterie an einem gemeinsamen, von einem Vorrat her beschickbaren Zulauf (23) und an gemeinsame Ablässe (35) angeschlossen sind.

# FIG.1

# FIG.2

0033724

FIG.3